# EUROPÄISCHE PATENTSCHRIFT

(11) **EP 1 469 797 B1**
(45) Veröffentlichungstag und Bekanntmachung des Hinweises auf die Patenterteilung: **02.11.2005**
(21) Anmeldenummer: 03790698.9
(22) Anmeldetag: 05.08.2003
(51) Int. Cl.: A61F 2/24

(54) **VORRICHTUNG ZUR IMPLANTATION UND BEFESTIGUNG VON HERZKLAPPENPROTHESEN**
DEVICE FOR THE IMPLANTATION AND FIXING OF HEART VALVE PROSTHESES
DISPOSITIF D'IMPLANTATION ET DE FIXATION DE PROTHESES DE VALVULES CARDIAQUES

(30) Priorität: 13.08.2002 DE 10237573
(43) Veröffentlichungstag der Anmeldung: 27.10.2004
(73) Patentinhaber: Figulla, Hans-Reiner, 07749 Jena (DE); Ferrari, Markus, 07747 Jena (DE); Fraunhofer-Gesellschaft zur Förderung der angewandten Forschung e.V., 80686 München (DE)
(72) Erfinder: FIGULLA, Hans-Reiner, 07749 Jena (DE); FERRARI, Markus, 07747 Jena (DE); GUYENOT, Volker, 07749 Jena (DE); DAMM, Christoph, 07743 Jena (DE); MÜLLER Ekkehard, 99425 Weimar (DE)
(74) Vertreter: Pfenning, Meinig & Partner GbR
(86) Internationale Anmeldenummer: PCT/DE2003/002669
(87) Internationale Veröffentlichungsnummer: WO 2004/019825

(56) Entgegenhaltungen:
- WO-A-02/24118
- US-A- 5 855 601
- US-A1- 2002 002 401
- US-A1- 2003 036 791

## Beschreibung

Die Erfindung betrifft eine Vorrichtung zur Implantation und Befestigung von Herzklappenprothesen mit einer an einer selbstexpandierbaren Verankerungsstütze befestigten Herzklappenprothese, wobei es sich bei der Herzklappenprothese um eine biologische oder mechanische Herzklappenprothese handeln kann.

Sie kann transluminal implantiert und sowohl für die Implantation einer Herzklappenprothese als Aorten- oder Pulmonalklappe eingesetzt werden.

Ausgehend von Herzklappenprothesenimplantationen, bei denen eine operative Öffnung des Brustkorbes von Patienten erforderlich war und während des Zeitraumes des operativen Eingriffes der jeweilige Patient an eine Herz-Lungen-Maschine angeschlossen werden musste, was für eine große Anzahl von Risikopatienten ausgeschlossen war und eine langwierige postoperative Behandlungsphase erforderte, wurde nach Lösungen gesucht, wie solche Implantationen minimal-invasiv erfolgen konnten.

Hierzu sind verschiedene Lösungen bekannt. So ist in DE 195 46 692 C2 eine selbstexpandierbare Herzklappenprothese zur Implantationen im menschlichen Körper über ein Kathetersystem mit einer Herzklappe und mit einem mit der Herzklappe verbundenen zusammenfaltbaren und expandierbaren Stent bekannt. Eine solche selbstexpandierbare Herzklappenprothese kann mit Hilfe eines Kathetersystems durch eine Leistenarterie bis hin zum Implantationsort am Herzen geführt werden. Nach Erreichen des Implantationsortes kann dann ein solcher Stent, der in seiner Längsrichtung aus mehreren relativ zueinander abwinkelbaren selbstexpandierenden Stent-Segmenten zusammengesetzt ist, sukzessive entfaltet werden. Nach der Entfaltung kann die Herzklappenprothese auch mit Unterstützung von Verankerungshaken zumindest im herznahen Bereich im jeweiligen Blutgefäß verankert werden.

Die eigentliche Herzklappenprothese befindet sich dabei unmittelbar im proximalen Bereich des Stents.

In DE 100 10 074 A1 ist eine Vorrichtung zur Befestigung und Verankerung von Herzklappenprothesen beschrieben, die im Wesentlichen aus drahtförmigen miteinander verbundenen Elementen gebildet ist. Dabei werden unterschiedliche Bügel eingesetzt, um eine sichere Befestigung und Abstützung einer Herzklappenprothese zu erreichen. Die darin beschriebene Vorrichtung verwendet drei jeweils gleiche Paare von Bügeln, die in jeweils einem Abstand von 120° zueinander angeordnet sind. Diese Bügel sind miteinander durch Festkörpergelenke verbunden, wobei die Festkörpergelenke die Funktion von Drehlagern erfüllen.

Zusätzlich sind entgegengesetzt gebogene Bügel vorhanden, die möglichst gleichlange Hebelarme bilden, um eine sichere Anlage von Bügeln auch bei peristaltischen Bewegungen am Herzen und Blutgefäß und eine sichere Abdichtung einer implantierten und fixierten Herzklappenprothese erreichen zu können.

Bei den bekannten Lösungen besteht jedoch die Gefahr einer Fehlimplantation von Herzklappen. Dies betrifft im Wesentlichen die exakte Positionierung und angulare Ausrichtung von zu implantierenden Herzklappenprothesen. Dabei ist mit den bekannten Lösungen die Entfernung einer nicht richtig positioniert implantierten Herzklappenprothese, wenn überhaupt nur sehr aufwendig möglich, wobei in diesem Fall zumindest ein weiterer operativer Eingriff erforderlich sein kann.

Solche Fehlimplantationen führen zu Undichtheiten (Klappeninsuffizienz) und dementsprechend erheblichen Belastungen des Ventrikels. Erfolgt beispielsweise eine Implantation einer Herzklappenprothese zu weit oberhalb der eigentlichen Herzklappenebene, kann es zum Verschluss der Abgänge der Herzkranzgefäße (Koronarien) und damit zu einer tödlichen Koronarischämie mit Herzinfarkt kommen.

Bei Implantationstechniken, bei denen Herzklappenprothesen bisher durch Leistenarterien minimal-invasiv bis an den Implantationsort am Herzen geführt worden sind, erfolgt die Einführung mittels eines Führungsdrahtes und mit Hilfe von Kathetern, wobei auch herkömmliche Ballonkatheter eingesetzt worden sind.

Bei einem solchen Eingriff wurde das Einführen durch Röntgendurchleuchtung (Herzkatheterlabor = HKL) und Ultraschall (transösophageales Echokardiogramm = TEE).

Trotz dieser Hilfsmittel kann jedoch nicht gesichert werden, dass mit den herkömmlichen Lösungen die erforderliche laterale Positionsgenauigkeit und insbesondere die angulare Lage von Herzklappenprothesen mit entsprechend daran befestigten Befestigungselementen erreicht werden konnte, wobei insbesondere ein entsprechender Winkelversatz eine Gefährdung für den jeweiligen Patienten wegen eines möglichen Verschlusses von Herzkranzgefäßen darstellen kann.

Insbesondere wirken an einer Aortenherzklappenprothese erhebliche Kräfte während der Füllungsphase des Herzzyklusses (Diastole), so dass eine sichere Verankerung erforderlich ist und ein Ablösen der Herzklappenprothese und Verankerungselementen verhindert werden muss.

Ausgehend hiervon ist es daher Aufgabe der Erfindung Möglichkeiten zu schaffen, um Herzklappenprothesen minimal-invasiv mit erhöhter Positioniergenauigkeit an einer Herzkammer eines Patienten implantieren zu können.

Erfindungsgemäß wird diese Aufgabe mit einer Vorrichtung, die die Merkmale des Anspruchs 1 aufweist, gelöst. Vorteilhafte Ausgestaltungsformen und Weiterbildungen der Erfindung können mit den in den untergeordneten Ansprüchen bezeichneten Merkmalen erreicht werden.

Die erfindungsgemäße Vorrichtung zur Implantation und Befestigung von Herzklappenprothesen mit einer an einer selbstexpandierbaren Verankerungsstütze (Stent) befestigten Herzklappenprothese ist dabei so ausgebildet, dass die Verankerungsstütze in gefalteter Form in einer mit einem Katheter verbindbaren Kartusche aufgenommen ist. Die Verankerungsstütze sollte dabei so komprimiert werden, dass sämtliche Teile ein gewisses Maß einer radial nach außen wirkenden Vorspannung aufweisen, die nach Freigabe durch eine Kartusche die Selbstexpansion bewirken.

Verankerungsstütze mit Herzklappenprothese können dann gemeinsam mit der Kartusche mit Hilfe eines Katheters durch eine Leistenaorta auf herkömmlichem Wege implantiert werden.

An der erfindungsgemäß einzusetzenden Verankerungsstütze sind proximalseitig Stützbügel ausgebildet. Diese Stützbügel werden dann während des eigentlichen Implantationsvorganges in die Taschen von Herzklappen des jeweiligen Patienten eingeführt, dabei bilden die Taschen der Herzklappe eines Patienten ein Widerlager, dass der proximalen Einführbewegung entgegenwirkt, so dass die Verankerungsstütze mit Herzklappenprothese lateral exakt positionierbar ist. Gleichzeitig wird gesichert, dass Verankerungsstütze und Herzklappenprothese eine exakte Winkellage einnehmen können, da hierfür die Taschen während des Einführens quasi eine Führung für die Stützbügel darstellen.

Die bezeichnenden Effekte können dadurch erreicht werden, dass durch eine vorgebbare erste Bewegung der Kartusche ausschließlich die Stützbügel der Verankerungsstütze frei gegeben werden und sich radial nach außen aufrichten und die weiteren Elemente der Verankerungsstütze innerhalb der Kartusche noch in gefalteter Form zusammengepresst gehalten werden.

Erst nachdem die Stützbügel in die Taschen der Herzklappe des jeweiligen Patienten eingeführt worden sind und die letztendliche Position für die Implantation erreicht worden ist, werden durch eine zweite Bewegung der Kartusche und/oder des eingesetzten Katheters die weiteren Elemente der Verankerungsstütze mit der Herzklappenprothese zu deren vollständiger Expansion und Verankerung der Verankerungsstütze frei gegeben und es wird eine sichere Befestigung der Herzklappenprothese exakt am günstigsten und besten Ort erreicht.

Fehlimplantationen, wie sie bei den bekannten Lösungen möglich waren, können so ausgeschlossen werden.

Des Weiteren besteht gegenüber den bekannten Lösungen aber auch die Möglichkeit, auch eine nicht zum Erfolg führende Implantation von Herzklappenprothesen abzubrechen und die Vorrichtung durch Zurückziehen des Katheters wieder zu entfernen, wobei hierfür bereits entfaltete Stützbügel wieder zusammengefaltet und in eine Kartusche oder ein Teil einer Kartusche rückgeführt werden können.

Die erfindungsgemäße Vorrichtung kann aber auch dadurch weitergebildet werden, dass an der Verankerungsstütze zusätzlich Kommissurenbügel vorhanden sind.

Dabei zählen die Kommissurenbügel zu Elementen der Verankerungsstütze, die erst bei einer zweiten nachfolgenden Bewegung der Kartusche und/oder des Katheters zu ihrer Expansion freigegeben werden, wenn die Stützbügel positionsrichtig in die Taschen einer Herzklappe eingeführt worden sind.

Beim Entfalten der Kommissurenbügel gelangen dann Teile der alten Herzklappe des jeweiligen Patienten zwischen jeweils einen Stütz- und einen Kommissurenbügel, so dass die jeweiligen Teile der alten Herzklappe zwischen diesen beiden Bügeln nach erfolgter Entfaltung der Verankerungsstütze klemmend gehalten werden, ähnlich wie es mit einem Papier zwischen den Bügeln einer Büroklammer der Fall ist.

Insbesondere für die nachfolgenden mittels Kartusche und Katheter und Betätigungselementen durchzuführenden Bewegungen, die zur sequentiellen Freigabe der einzelnen Elemente der Verankerungsstütze führen, ist es vorteilhaft, eine mehrteilige Kartusche einzusetzen, wobei mindestens zwei einzelne Teile jeweils relativ zueinander bewegt werden können.

So können beispielsweise die für die Selbstexpansion durchzuführenden Bewegungen einer Kartusche oder einzelner Teile einer Kartusche ein proximales und/oder distales Verschieben sein, das in mehreren nacheinander durchzuführenden Stufen erfolgen kann, wobei jeweils unterschiedliche Wege zurückgelegt werden, um die entsprechenden Teile zum jeweiligen Zeitpunkt einer Implantation für deren Expansion nacheinander frei zugeben.

So kann beispielsweise die erste Bewegung ein distales Zurückziehen der Kartusche oder eines Teiles einer Kartusche sein.

In diesem Fall kann dann, falls dies erforderlich sein sollte, um eine Fehlimplantation zu vermeiden eine proximale Bewegung der Kartusche oder eines Teils einer Kartusche dazu benutzt werden, um die bereits entfalteten radial nach außen mit einer Vorspannkraft wirkenden Stützbügel wieder zusammenzufalten und im Inneren der Kartusche aufzunehmen, so dass eine Entfernung der Vorrichtung aus dem Patienten möglich ist.

Als Betätigungselemente für eine Manipulation und dementsprechend eine Verschiebungsbewegung der Kartusche oder einzelner Teile der Kartusche können Bowdenzüge oder flexible Schubrohre eingesetzt werden, die durch das Innere des Katheters zur Kartusche oder einem Teil der Kartusche geführt sind, eingesetzt werden.

Solche Betätigungselemente können aber auch an Befestigungselementen, beispielsweise Ösen, die an der Verankerungsstütze vorhanden sind, angreifen.

Solche Befestigungselemente können aber auch allein oder zusätzlich für eine Fixierung der Verankerungsstütze am jeweiligen Blutgefäß benutzt werden.

Um die zeitversetzte Entfaltung von Stützbügeln zu ermöglichen, können an der Kartusche Schlitze ausgebildet sein, durch die nach Initialisierung der ersten Bewegung der Kartusche eine Entfaltung der Stützbügel möglich wird.

Solche Schlitze können in unterschiedlichster Gestalt, ausgehend von einer Stirnseite an der Kartusche in gerader Form, in schräg geneigten Winkeln oder in einer Kurvenform (Wellenform) ausgebildet sein.

Die Freigabe der Stützbügel zu deren Entfaltung kann dabei durch in proximaler Richtung offene Schlitze einer Kartusche oder eines Teiles einer Kartusche erfolgen, wenn die Kartusche oder ein entsprechendes Teil einer Kartusche in eine distale Richtung bewegt, also zurückgezogen wird oder beispielsweise auch eine Drehbewegung um die Längsachse der Kartusche durchgeführt werden kann, so dass die jeweiligen Schlitze in Bezug zu den Stützbügeln so ausgerichtet werden, dass sich die Stützbügel durch die freien Schlitze entfalten können.

Solche Schlitze können aber auch temporär durch ein weiteres Teil einer Kartusche abgedeckt gehalten sein, bis durch eine entsprechende Relativbewegung der einzelnen Kartuschenteile in proximaler bzw. distaler Richtung oder eine Drehung um die Längsachse die Schlitze freigegeben werden und die Stützbügel entfaltet werden können.

Hierbei können an dem Teil einer Kartusche, an dem keine Schlitze ausgebildet sind, diese Schlitze überdeckende Stege ausgebildet sein, die eine Freigabe der Stützbügel bis zum Einführen in die Taschen in die Herzklappe eines Patienten verhindern und die Freigabe der Stützbügel zu deren Entfaltung erst nach entsprechender Relativbewegung der jeweiligen Teile einer Kartusche erfolgen kann.

Die an einer Verankerungsstütze vorhandenen Stützbügel und auch die Kommissurenbügel sollten in proximaler Richtung konvex und bogenförmig gekrümmt sein und durch eine solche abgerundete Form können Verletzungen des Blutgefäßes am Herz vermieden sowie das Entfalten bei der Selbstexpansion erleichtert werden. Ebenfalls können die Stützbügel mit einer solchen Gestaltung entsprechend einfacher in die Taschen der alten Herzklappe eingeführt werden, ohne dass entsprechende Verletzungen am Gewebe oder dort vorhandenen Blutgefäßen zu verzeichnen sind.

An Verankerungsstützen können aber auch zusätzliche Stabilisierungsbügel vorhanden sein, mit denen eine Erhöhung der Festigkeit nach der Selbstexpansion der verankerten Verankerungsstütze erreichbar ist.

Solche Stabilisierungsbügel können vorteilhaft sein, da für die Ausnutzung des erforderlichen Selbstexpansionseffektes an einer Verankerungsstütze für eine sichere Fixierung der Verankerungsstütze mit der Herzklappenprothese, unter Berücksichtigung der Tatsache, dass während der Phase des Einführens einer innerhalb einer Kartusche gefalteten Verankerungsstütze möglichst kleine Volumina erreicht werden müssen, kleine Querschnitte für die jeweiligen Bügel eingehalten werden müssen.

Sämtliche Bügel einer Verankerungsstütze sollten dabei so angeordnet, gestaltet und dimensioniert sein, dass die nacheinander erfolgende Freigabe von Stützbügeln und den anderen Bügeln mit gegebenenfalls weiteren an einer Verankerungsstütze vorhandenen Elementen und der Herzklappenprothese durch entsprechende Manipulation von Kartusche und/oder Katheter erreicht werden können. Dabei sollte selbstverständlich auch die Gestaltung der Kartusche oder mindestens eines Teiles einer Kartusche berücksichtigt sein, was beispielsweise auf die bereits angesprochenen Schlitze und die diese Schlitze überdeckenden Stege zutreffen kann.

Entsprechend der Anatomie sollten an der Verankerungsstütze drei in jeweils gleichen Winkelabständen zueinander angeordnete Stützbügel vorhanden sein.

Dementsprechend können auch drei Kommissurenbügel vorhanden sein, wobei die Mindestzahl von an einer Verankerungsstütze vorhandenen Kommissurenbügeln jedoch zwei betragen sollte.

Die Stütz- und die Kommissurenbügel können dabei an einer Verankerungsstütze in jeweils gleicher Winkellage angeordnet sein, so dass jeweils ein Stützbügel in jeweils eine Tasche der alten Herzklappe eingeführt und vom entsprechenden Kommissurenbügel dann der Teil der Herzklappe in Verbindung mit dem Stützbügel klemmend vom Kommissurenbügel gehalten wird.

Es besteht aber auch die Möglichkeit, dass die an einer Verankerungsstütze vorhandenen Stütz- und Kommissurenbügel jeweils in einem Winkelversatz zueinander angeordnet sind.

In diesem Fall sind dann im implantierten Zustand die Stützbügel mit ihren proximalen Teilen in die Taschen einer alten Herzklappe eingeführt und die alte Herzklappe kann dann so klemmend mit Stützbügeln gehalten werden, dass ein Kommissurenbügel mit zwei benachbarten Stützbügeln zwei Teile der alten Herzklappe klemmend hält.

Die Stabilität einer implantierten und befestigten Verankerungsstütze kann mittels mindestens eines Ringträgers, der ein Element an einer Verankerungsstütze sein kann, erhöht werden.

So besteht die Möglichkeit die an einer Verankerungsstütze vorhandenen unterschiedlichen Bügel, bevorzugt an deren Fußpunkten mit einem solchen Ringträger zu verbinden. Dabei ist es nicht zwingend erforderlich, eine Verbindung zwischen Ringträger und sämtlichen Bügeln einer Verankerungsstütze vorzusehen.

Eine Verankerungsstütze mit einem zusätzlichen Ringträger kann aber auch so ausgebildet sein, dass ein Ringträger in einem Abstand zur Ebene, in der die Herzklappenprothese an der Verankerungsstütze fixiert ist, beispielsweise mit Längsstegen angeordnet ist. Ein solcher Ringträger sollte dann eine Stützstruktur aufweisen, die z-förmig, wellenförmig oder mäanderförmig ist.

Die erfindungsgemäße Vorrichtung kann auch gemeinsam mit einem Ballonkatheter eingesetzt werden. Mit dem Ballonkatheter kann die alte Herzklappe vor der Selbstexpansion der Verankerungsstütze weg gedrückt werden.

Nachfolgend soll die Erfindung beispielhaft näher erläutert werden.

Dabei zeigen:
- Figur 1: in schematischer Form ein Beispiel einer implantierten Verankerungsstütze; gezeigt wird eine Ausarbeitung des Stents als Verankungsbügel/-haken zur mechanischen Stabilisierung und exakten Positionierung des Herzklappen tragend Stents,
- Figur 2: in schematischer Form eine Implantation einer Verankerungsstütze mit Herzklappenprothese;
- Figuren 3a bis 3d: vier zeitlich nachfolgende Phasen bei einer Implantation einer Herzklappenprothese;
- Figur 4: in perspektivischer Darstellung ein Beispiel für eine Verankerungsstütze;
- Figur 5: ein weiteres Beispiel einer Verankerungsstütze;
- Figur 6: ein weiteres Beispiel für eine Verankerungsstütze;
- Figur 7: ein weiteres Beispiel für eine Verankerungsstütze;
- Figur 8: ein weiteres Beispiel für eine Verankerungsstütze;
- Figur 9: ein weiteres Beispiel für eine verankerungsstütze;
- Figur 10: eine Darstellung einer teilentfalteten Verankerungsstütze innerhalb einer Kartusche;
- Figur 11: eine weiter teilentfaltete Verankerungsstütze in einer Kartusche nach Figur 10;
- Figur 12: eine voll selbstexpandiert entfaltete Verankerungsstütze außerhalb einer Kartusche nach Figuren 10 und 11;
- Figur 13: ein Beispiel einer mehrteiligen Kartusche;
- Figur 14: eine teilentfaltete Verankerungsstütze in einer Kartusche nach Figur 13;
- Figur 15: eine vollentfaltete Verankerungsstütze außerhalb einer Kartusche nach den Figuren 13 und 14;
- Figur 16: ein weiteres Beispiel einer Kartusche;
- Figur 17: eine Kartusche nach Figur 16 mit teilentfalteter Verankerungsstütze;
- Figur 18: eine Kartusche nach den Figuren 16 und 17 mit weiter entfalteter Verankerungsstütze;
- Figur 19: eine vollentfaltete Verankerungsstütze außerhalb einer Kartusche nach den Beispielen gemäß Figuren 16 bis 18 und
- Figur 20: ein weiteres Beispiel einer mehrteiligen Kartusche.

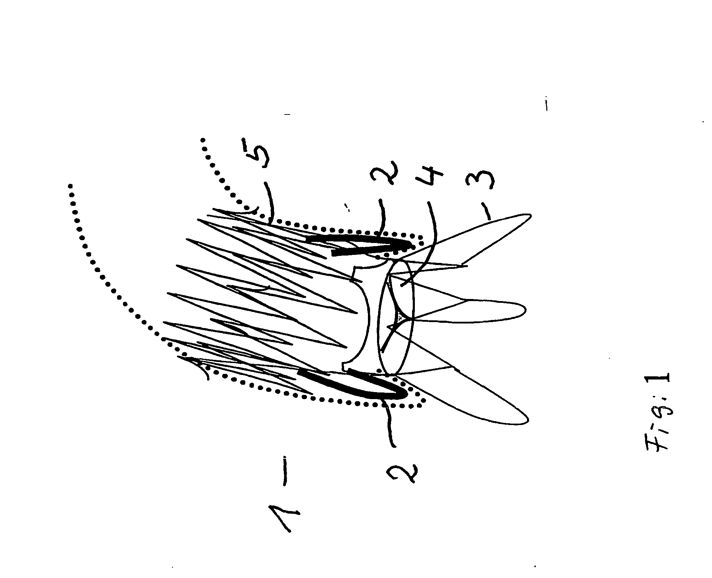

Figur 1 zeigt in einer schematischen Darstellung ein Beispiel einer implantierten Verankerungsstütze 1 mit einer daran aufgespannten Herzklappenprothese 4.

Dabei sind an der hier gezeigten Verankerungsstütze 1 Stützbügel 2 vorhanden, die nach dem Einführen in die Taschen der alten Herzklappe des jeweiligen Patienten eingreifen.

Außerdem sind an der Verankerungsstütze 1 hier drei Kommissurenbügel 3 vorhanden, die ebenfalls nach der Expansion radial nach außen wirkende Kräfte ausüben und dabei gleichzeitig neben einer gewissen Verankerungswirkung für die Verankerungsstütze 1 Teile der alten Herzklappe des Patienten, die zwischen den Kommissurenbügeln 3 und den Stützbügeln 2 angeordnet sind, klemmend halten.

Bei dem hier gezeigten Beispiel einer Verankerungsstütze 1 weist diese distalseitig einen Ringträger 5, der als z-förmige Stützstruktur ausgebildet ist, auf. Auch diese Stützstruktur weist selbstexpandierende Eigenschaften auf.

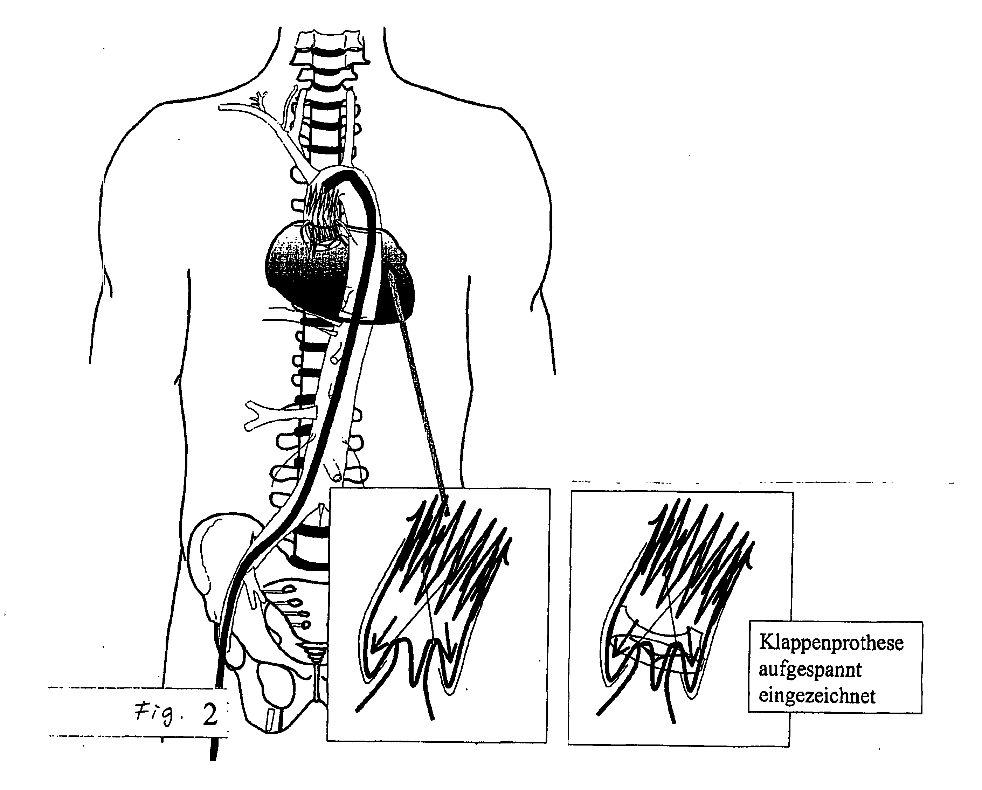

Mit Figur 2 soll in schematischer Form verdeutlicht werden, wie eine Verankerungsstütze 1 mit Herzklappenprothese 4 transvaskulär durch die Leistenaorta implantiert werden kann.

Mit den beiden zusätzlichen Ausschnitten, wie sie in Figur 2 gezeigt sind, soll nochmals verdeutlicht werden, wie Stützbügel 2 einer Verankerungsstütze 1 in die Taschen einer alten Herzklappe eines Patienten eingeführt werden können und nachfolgend nach vollständiger Expansion/Entfaltung der Verankerungsstütze 1 ein Aufspannen der Herzklappenprothese 4 erreichbar ist. Es wird außerdem gezeigt, wie die Bügel des Stents der Verankerung und sicheren Plazierung der Klappen -Stent-Prothese in den Taschen der alten zu ersetzenden Klappe dienen.

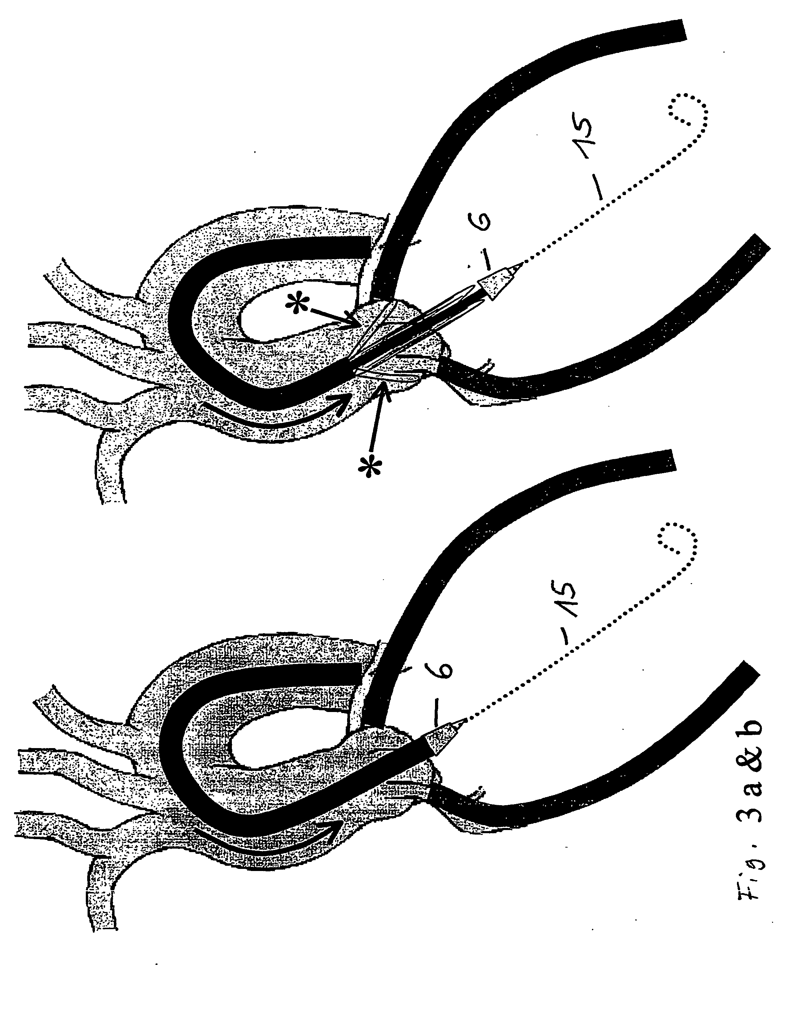

In den Figuren 3a bis 3d sind mehrere zeitlich nachfolgende Phasen bei der transvaskulären Implantation einer Herzklappenprothese dargestellt.

Figur 3a zeigt das transvaskuläre Einführen einer Verankerungsstütze 1 entlang eines Führungsdrahtes 15, die in einer Kartusche 6 auf einem relativ kleinen Volumen zusammengefaltet ist, wobei Kartusche 6 und ein hier nicht explizit dargestelltes Katheter äußere Abmessungen aufweist, die kleiner als der Innendurchmesser des für die transvaskuläre Implantation genutzten Blutgefäßes ist.

Das Einführen der Kartusche 6 mit Verankerungsstütze 1 kann hierbei in üblicher Form mittels Röntgenstrahlung und Ultraschalltechnik kontrolliert werden.

Erreicht dabei die Kartusche 6 in etwa die Position für die eigentliche Implantation wird an der Kartusche 6 oder auch mittels eines Katheters eine definiert vorgebbare Bewegung ausgeführt, die, wie dies in Figur 3b verdeutlicht ist, zur Freigabe und Entfaltung der Stützbügel 2 führt und diese sich dabei radial nach außen entfalten. Daraufhin wird die ganze Vorrichtung durch das jeweilige Blutgefäß, üblicherweise die Leistenaorta oder die Arteria pulmonalis in proximaler Richtung verschoben, so dass die Stützbügel 2 die alte Herzklappe des jeweiligen Patienten hintergreifen und in die Taschen dieser Herzklappe eingeführt werden. Berühren die Stützbügel 2 mit ihren proximalen Stirnseiten die Fußpunkte der Taschen der alten Herzklappen ist ein spürbarer Widerstand zu verzeichnen.

Nachfolgend kann mit der Kartusche 6 oder einem Katheter mindestens eine weitere Bewegung durchgeführt werden, die weitere Elemente der Verankerungsstütze 1 freigibt und zu deren radial nach außen gerichteten Entfaltung führt.

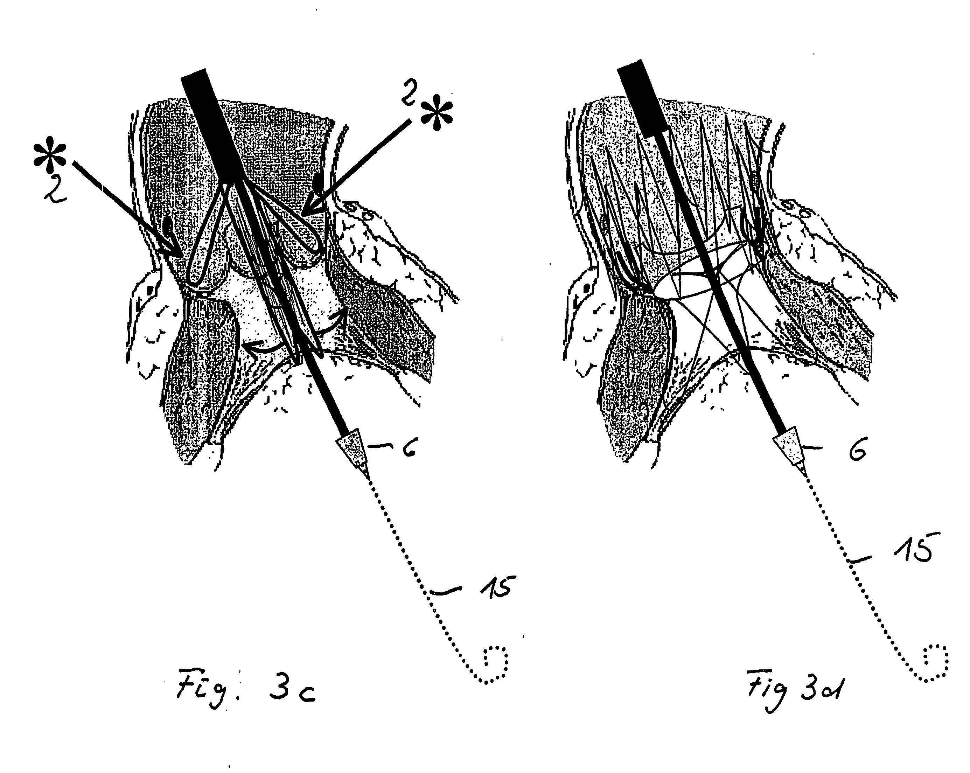

So werden, wie dies mit Figur 3c verdeutlicht wird, durch entsprechende Relativbewegung von Kartusche 6 und Verankerungsstütze 1 Kommissurenbügel 3 für die Entfaltung aus der Kartusche 6 radial nach außen zeitversetzt erst dann freigegeben, wenn die Stützbügel 2 in die Taschen der alten Herzklappe des jeweiligen Patienten eingeführt worden sind. Dadurch ist die gesamte Verankerungsstütze 1 mit allen ihren Elementen sowohl in lateraler Richtung, wie auch in ihrer Winkellage genau positioniert und durch die vollständige Entfaltung der Verankerungsstütze 1, nach deren vollständiger Entfaltung, wie dies in Figur 3d dargestellt ist, kann eine sichere Befestigung der Verankerungsstütze 1 mit Herzklappenprothese 4 erreicht werden, und nachfolgend der Katheter mit der Kartusche 6 durch Herausziehen wieder entfernt werden.

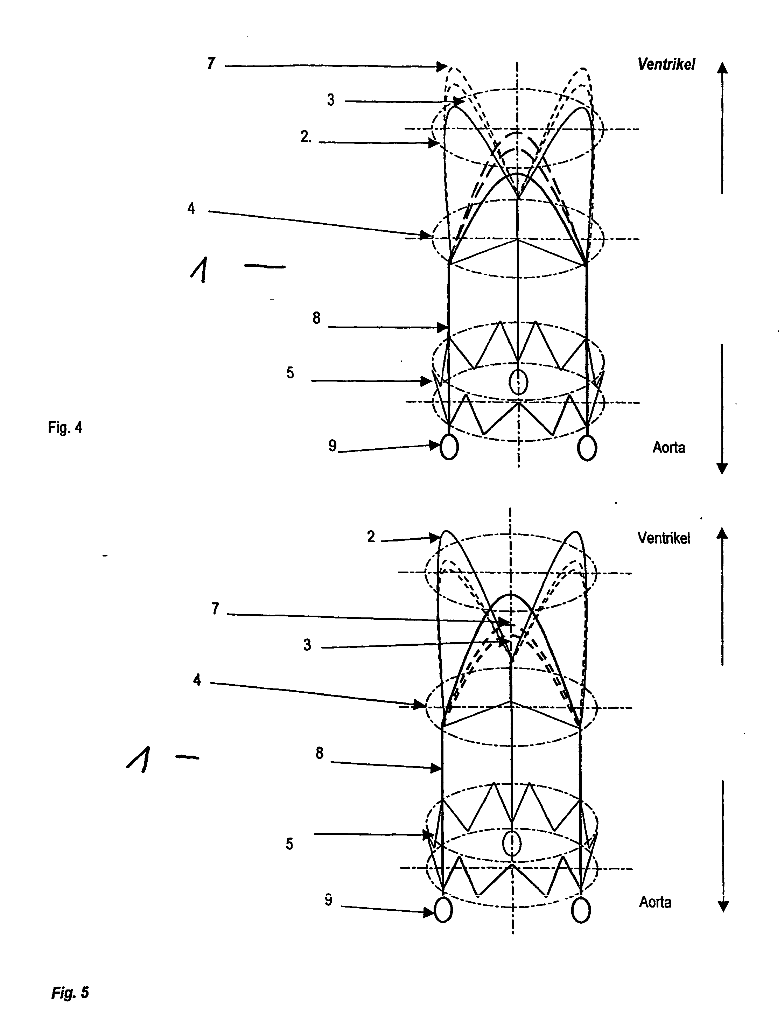

Mit den Figuren 4 bis 9 sollen einige modifizierte Beispiele von Verankerungsstützen 1, die bei der Erfindung eingesetzt werden können, weiter erläutert werden. Dabei sind gleiche Elemente mit den jeweils gleichen Bezugszeichen versehen worden.

Die Verankerungsstütze 1, wie sie in Figur 4 dargestellt ist, weist neben den drei erfindungswesentlichen Stützbügeln 2, in proximaler Richtung verlängerte Kommissurenbügel 3 sowie entsprechende Stabilisierungsbügel 7 auf, die sämtlichst in proximaler Richtung konvex gewölbt ausgebildet sind. Die Fußpunkte der Stütz-, Kommissuren- und Stabilisierungsbügel 2, 3 und 7 sind bei diesem und den nachfolgend noch zu beschreibenden Beispielen im Wesentlichen in der Ebene, in der auch die Herzklappenprothese 4 angeordnet ist, angeordnet.

Im Gegensatz zum Beispiel nach Figur 4, bei dem die Stabilisierungsbügel 7 in proximaler Richtung länger als die Kommissurenbügel 2 und die wiederum etwas länger als die Stützbügel 2 ausgebildet sind, ist beim Beispiel nach Figur 5 eine verlängerte Ausbildung von Stützbügeln 2 gegenüber der Länge von Stabilisierungsbügeln 7 und Kommissurenbügeln 3, deren Länge sich wiederum nur geringfügig voneinander unterscheidet, gewählt worden.

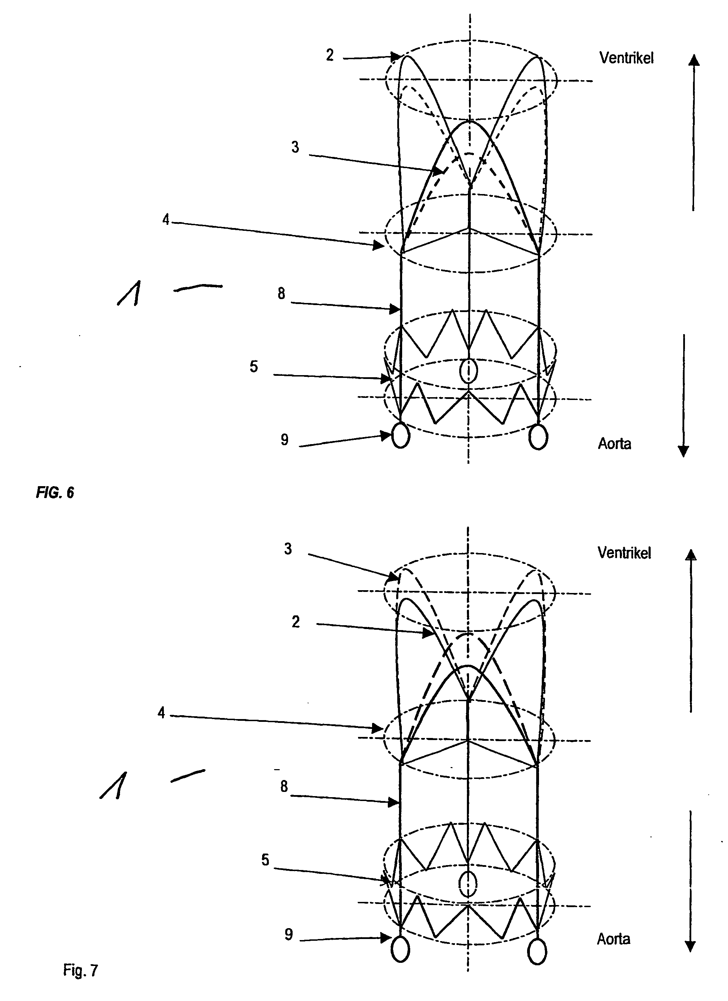

Das in Figur 6 gezeigte Beispiel entspricht im Wesentlichen dem Beispiel nach Figur 5. Es wurde jedoch auf Stabilisierungsbügel 7 verzichtet.

Beim Beispiel nach Figur 7 wurden wieder gegenüber den Stützbügeln 2 in proximaler Richtung verlängerte Kommissurenbügel 3 eingesetzt.

Bei den in den Figuren 4 bis 7 gezeigten Beispielen für Verankerungsstützen 1 sind zusätzliche jeweils gleiche Elemente an einer Verankerungsstütze 1 eingesetzt worden.

So sind jeweils Ringträger 5 über in Längsrichtung der Verankerungsstütze 1 ausgerichtete Stege 8 jeweils mit Fußpunkten der jeweiligen Bügel 2, 3 bzw. 7 verbunden.

Ein solcher Ringträger 5 ist vorteilhaft in Form einer selbstexpandierbaren Stützstruktur, hier bei dem gezeigten Beispiel eine z-förmige Stützstruktur ausgebildet, die vorteilhaft durch radial nach außen wirkende Anpressdrücke und ihre Gestaltung eine weiter verbesserte Fixierung der Verankerungsstütze 1 am Implantationsort bewirkt, so dass ein Verschieben oder ein Verdrehen der Verankerungsstütze 1 mit Herzklappenprothese 4 zusätzlich verhindert werden kann.

Des Weiteren sind an der Verankerungsstütze 1 distalseitig Befestigungselemente 9, hier in Form von Ösen, ausgebildet. An diesen Befestigungselementen 9 können Manipulationselemente angreifen, so dass ein Verschieben oder auch ein Verdrehen der Verankerungsstütze 1 dadurch erleichtert werden kann.

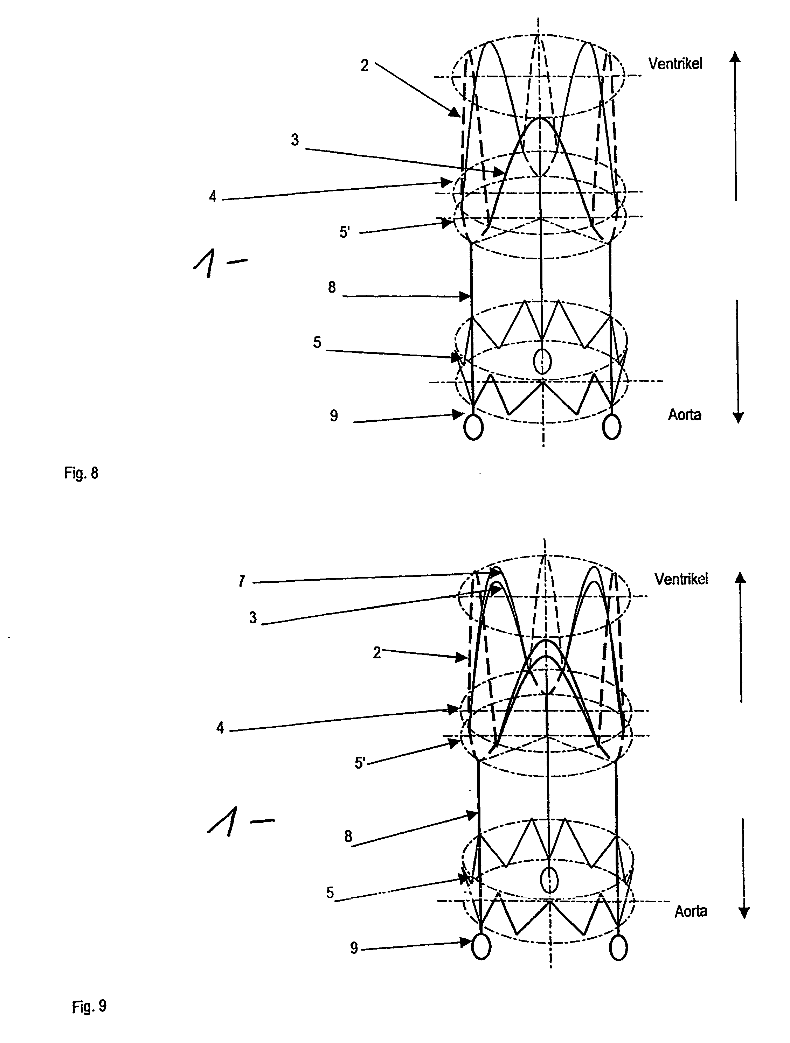

Beim Beispiel nach Figur 8 sind Stützbügel 2 und Kommissurenbügel 3 mit jeweils gleicher Länge in proximaler Richtung eingesetzt worden. Die Stützbügel 2 sind jedoch in einem Winkelversatz zu den Kommissurenbügeln 3 angeordnet.

Der Winkelversatz und die Gestaltung mit Dimensionierung der Kommissurenbügel 3 sollte aber so gewählt sein, das Teile der alten Herzklappe eines Patienten gemeinsam mit in den Taschen der alten Herzklappe eingeführten Stützbügeln 2 klemmend gehalten werden können. So kann es beispielsweise vorkommen, dass ein Kommissurenbügel 3 Teile zweier alter Herzklappenteile eines Patienten mit jeweils benachbart zu diesem Kommissurenbügel 3 angeordneten Teilen von Stützbügeln 2 klemmend hält.

Bei dem in Figur 9 gezeigten Beispiel wurde wieder eine mit einem bestimmten vorgegebenen Winkelversatz gewählte Anordnung von Stützbügeln 2 und Kommissurenbügeln 3 gewählt. Dabei sind aber die Stützbügel 2 in proximaler Richtung deutlich kürzer als die Kommissurenbügel 3 und es wurden zusätzliche Stabilisierungsbügel 7, die in gleicher Winkellage mit den Kommissurenbügeln 3 angeordnet sind, vorgesehen. Dabei unterscheiden sich die Kommissurenbügel 3 und die Stabilisierungsbügel 7 in ihrer Länge in proximaler Richtung nur geringfügig.

An den in den Figuren 8 und 9 gezeigten Beispielen sind zusätzliche Ringträger 5' vorhanden, an denen die Fußpunkte sämtlicher Bügel 2, 3 und 7 angeordnet sind und angreifen können. Die Herzklappenprothese 4 kann dann in einer Ebene im Bereich des Ringträgers 5' angeordnet sein.

Auch bei den in den Figuren 8 und 9 gezeigten Beispielen ist ein weiterer Ringträger 5 über Stege 8 mit der Verankerungsstütze 1 verbunden.

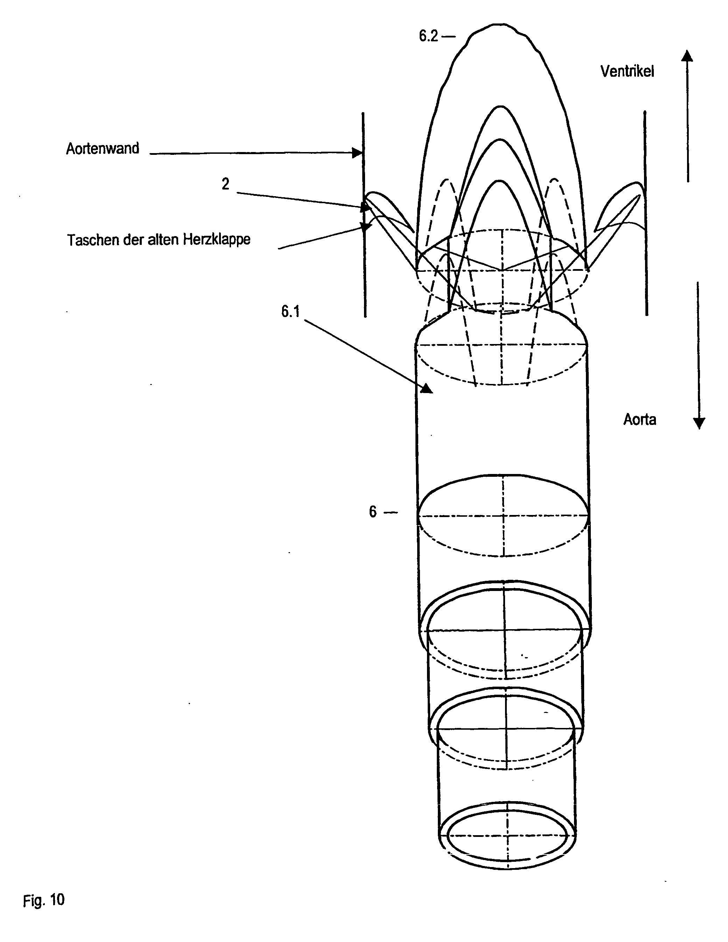

Nachfolgend sollen mittels der Figuren 10 bis 20 beispielhaft Gestaltungsmöglichkeiten und Funktionen von Kartuschen 6, die bei erfindungsgemäßen Vorrichtungen eingesetzt werden können, erläutert werden.

Dabei handelt es sich generell um mehrteilige Kartuschen 6. Die einzelnen Teile der Kartuschen können dann relativ zueinander bewegt werden, um eine sequentielle Freigabe und Entfaltung von Elementen von Verankerungsstützen 1 zu ermöglichen.

In Figur 10 ist eine Kartusche 6 gezeigt, an der ein proximales Endteil 6.2 während des Einführvorganges der Verankerungsstütze 1 zumindest die größten Teile von Bügeln 2, 3 und 7 aufnehmen kann und weitere Elemente der Verankerungsstütze 1 in zusammengefalteter Form in einem Mittelteil 6.1 der Kartusche 6 aufgenommen sind.

In Figur 10 ist bereits eine Phase einer Implantation einer Verankerungsstütze 1 dargestellt, bei der durch eine Relativbewegung der Teile 6.1 und 6.2 eine Freigabe von Stützbügeln 2 zu deren radial nach außen gerichteter Entfaltung erfolgt ist und die Stützbügel 2 bereits in Taschen einer alten Herzklappe eingeführt worden sind. Die Aortenwandung ist hier schematisch angedeutet.

Die übrigen Bügel sind noch innerhalb des proximalen Teiles 6.2 der Kartusche und mit ihren Fußpunkten im Mittelteil 6.1 zusammengedrückt in gefalteter Form aufgenommen.

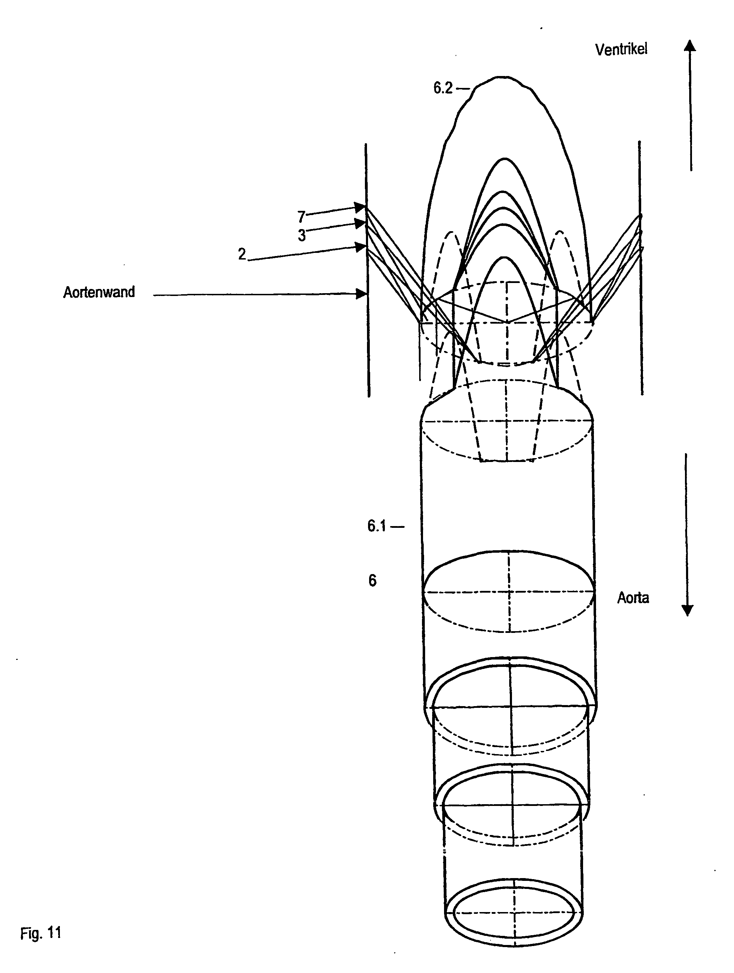

In einem zweiten Bewegungsschritt, der eine weiterführende Relativbewegung zwischen den Teilen 6.1 und 6.2 der Kartusche 6 darstellt, wurden auch die Stabilisierungsbügel 7 und Kommissurenbügel 3 einer Verankerungsstütze 1 freigegeben und konnten sich in radialer Richtung nach außen entfalten, wie dieses Implantationsphasenstadium mit Figur 11 verdeutlicht werden soll.

Dabei befinden sich Teile der alten Herzklappe des Patienten zumindest zwischen Stützbügeln 2 und Kommissurenbügeln 3 und durch die radial nach außen wirkenden Kräfte der Kommissurenbügel 3 und die Abstützwirkung der Aortenwand werden die alten Teile der Herzklappe eines Patienten zumindest zwischen Stützbügeln 2 und Kommissurenbügeln 3 eingeklemmt. Die Stabilisierungsbügel 7 können diese Klemmwirkung gegebenenfalls unterstützen.

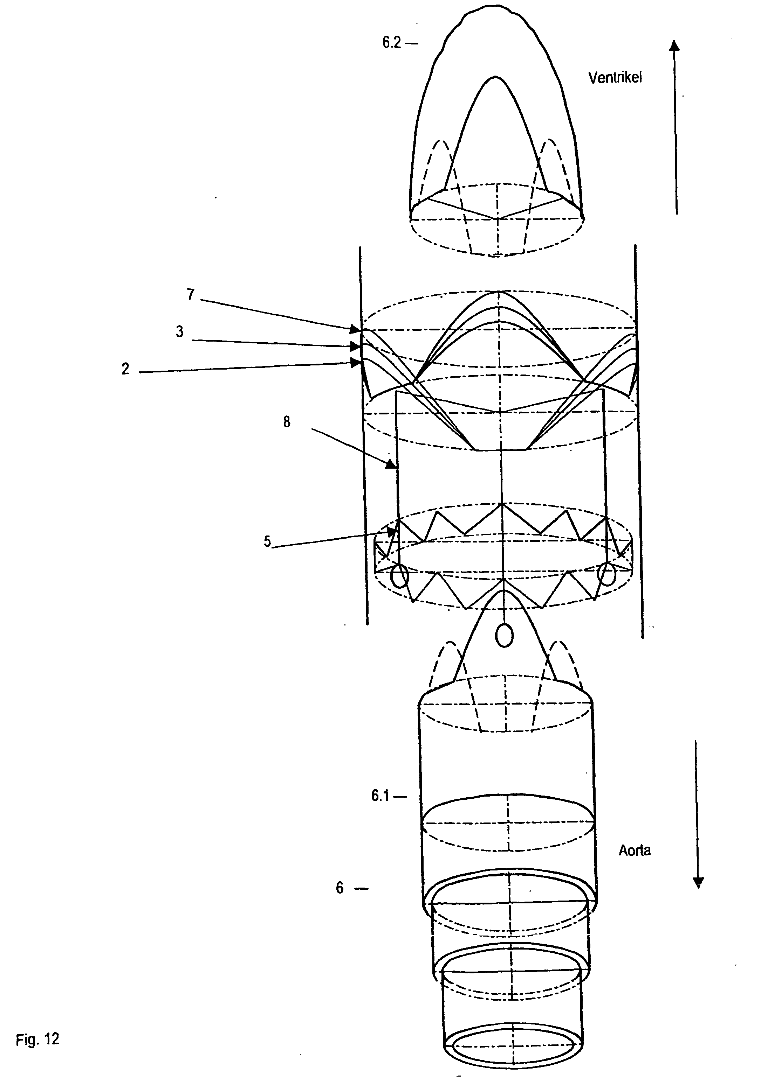

In Figur 12 ist ein wiederum zu Figur 11 nachfolgendes Stadium bei einer Implantation einer Herzklappenprothese 4 dargestellt. Durch ein Zurückziehen der distal angeordneten Teile der Kartusche 6 und hier insbesondere zumindest dem Mittelteil 6.1 wird die gesamte Verankerungsstütze 1 freigegeben und kann sich selbst expandierend entfalten, wobei gleichzeitig die jeweilige Herzklappenprothese 4 unabhängig davon, ob es sich um ein künstliches oder biologisches Implantat handelt, entfaltet wird.

Nachfolgend können sämtliche Teile der Kartusche entfernt werden und die Herzklappenprothese 4 sofort ihre Funktion aufnehmen.

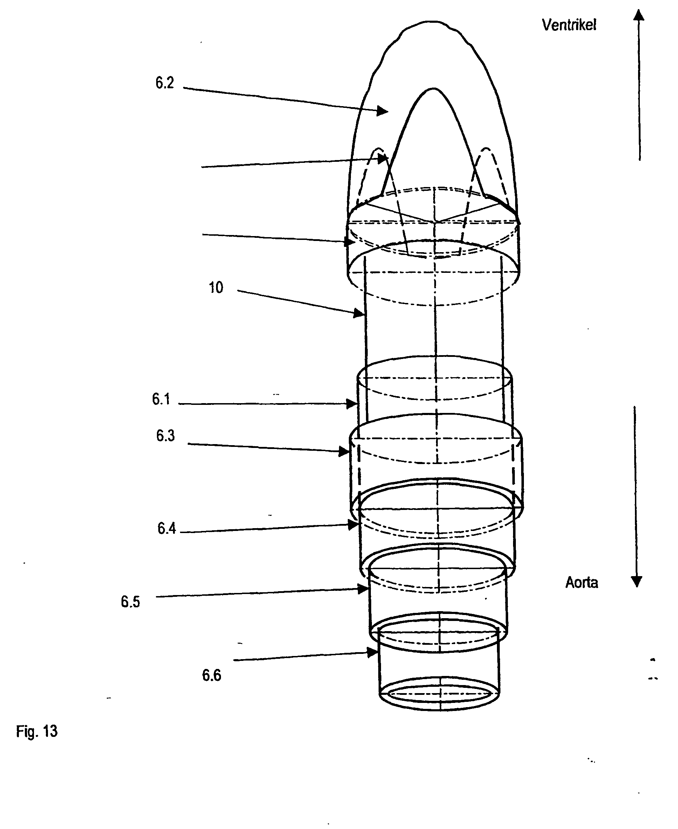

In Figur 13 ist wiederum eine Kartusche 6 mit mehreren einzelnen Teilen 6.1 bis 6.6 dargestellt, die eine zusammengefaltete Verankerungsstütze 1 aufnimmt.

Auch hier ist ein proximales Teil 6.2 vorhanden, das im Wesentlichen die vorderen Teile von Stützbügeln 2 aufnehmen muss.

Am proximalen Teil 6.2 greifen Betätigungselemente 10, bei diesem Beispiel Bowdenzüge zu dessen Manipulation und zur Auslösung der für die Freigabe von Bügeln erforderlichen Relativbewegung an.

Das Mittelteil 6.1 nimmt die weiteren Elemente einer Verankerungsstütze 1 im Wesentlichen auf.

Das sich in distaler Richtung daran anschließende Teil 6.3 dient im Wesentlichen als Aufnahmeelement für Betätigungselemente 10 für die Kartuschenteile 6.1 und 6.2.

Die Teile 6.4, 6.5 und 6.6 nehmen wiederum Betätigungselemente durch ihre innere hohle Gestaltung auf und ermöglichen durch ihre teleskopförmige Anordnung und Gestaltung eine Einflussnahme auf die für die Implantation der Verankerungsstütze 1 mit Herzklappenprothese 4 erforderlichen sequentiell durchzuführenden Relativbewegungen.

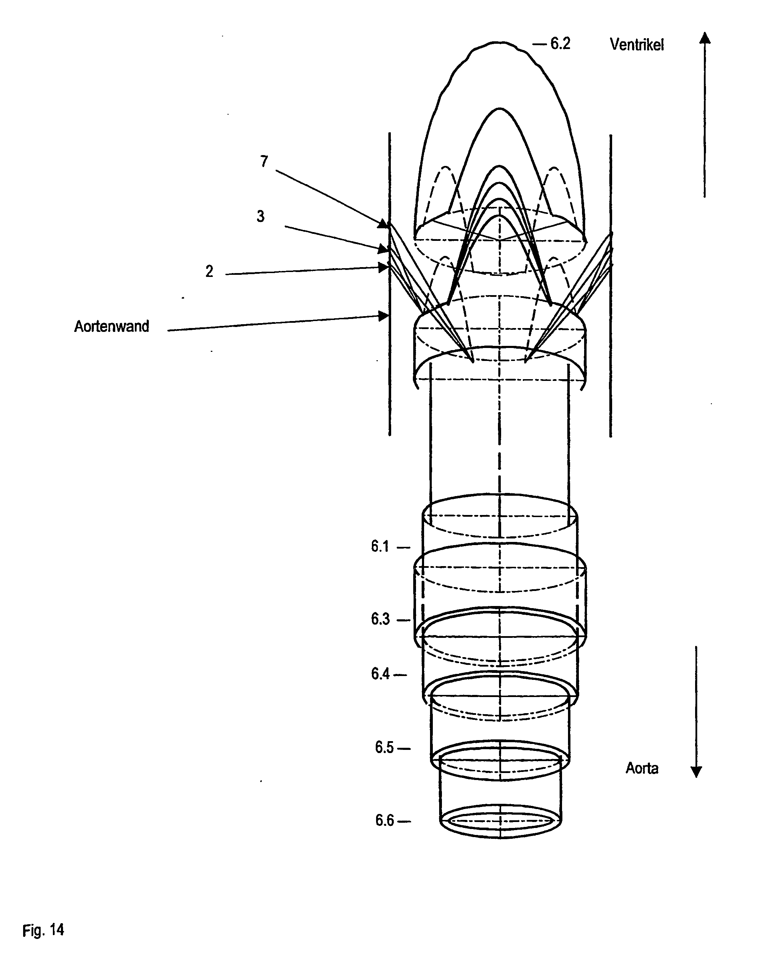

Figur 14 zeigt wiederum eine nachfolgende Phase bei einer Implantation einer Herzklappenprothese 4 mit einer Verankerungsstütze 1, bei der wiederum durch entsprechende Relativbewegung der einzelnen Elemente 6.1 bis 6.6 der hier mehrteiligen Kartusche 6 eine sequentielle Freigabe und Entfaltung der einzelnen Stützbügel 2, nachfolgend der Kommissurenbügel 3 und Stützbügel 7 für die exakte Positionierung mit Ausrichtung und dem Einklemmen der Teile einer alten Herzklappe eines Patienten realisiert worden ist.

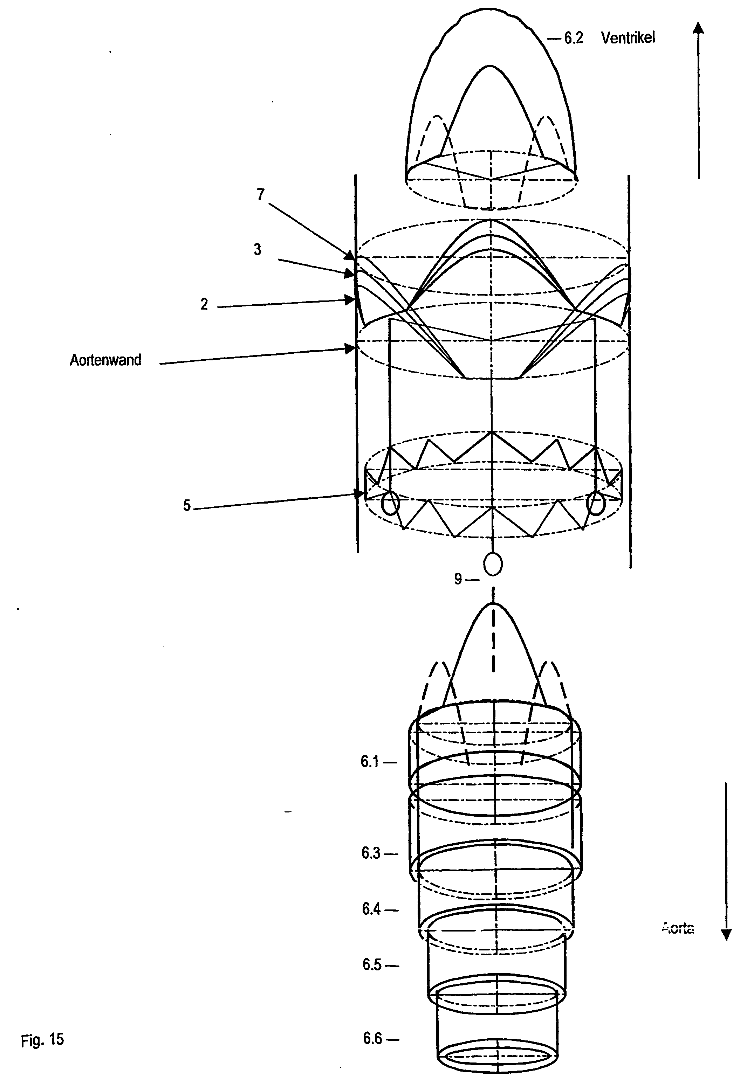

Figur 15 zeigt dann wiederum eine voll entfaltete Verankerungsstütze 1 nach entsprechend weiterer Relativbewegung der einzelnen Elemente 6.1 bis 6.6 der Kartusche 6, wobei das proximale Teil 6.2 von den übrigen Teilen der Kartusche getrennt worden ist und gesondert entfernt werden muss.

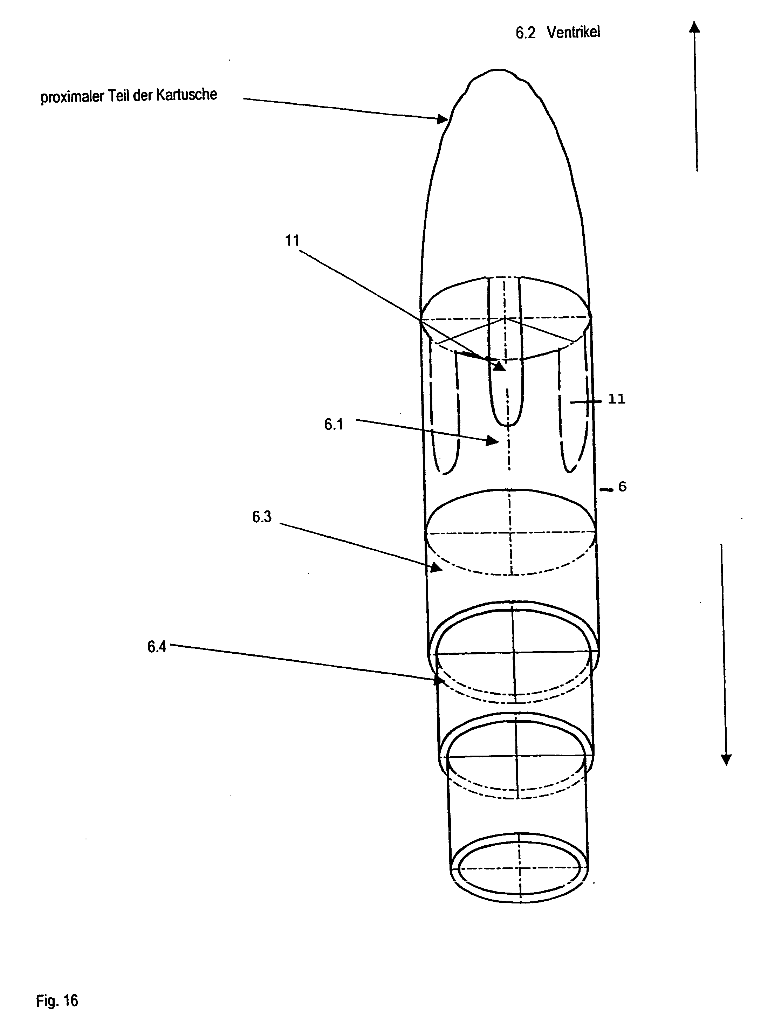

Auch in Figur 16 ist wiederum eine mehrteilige Kartusche 6 dargestellt. Dabei sind bei dem hier gezeigten Beispiel einer Kartusche 6 am proximalen Teil 6.2 der Kartusche 6 in distaler Richtung überstehende Stege 11 vorhanden. Die Stege 11 sind dabei so angeordnet und dimensioniert, dass durch eine entsprechende Relativbewegung zumindest der Teile 6.1 und 6.2, die bei einer Implantation durchzuführende zeitversetzte Freigabe von Stützbügeln 2 und zumindest Kommissurenbügeln 3 erreicht werden kann.

Dies kann beispielsweise in nicht dargestellter Form durch eine Verdrehung um die Längsachse der Kartusche 6 des proximalen Teiles 6.2 erreicht werden, so dass hier ebenfalls nicht dargestellte Schlitze am Teil 6.1 freigegeben werden und durch die freigegebenen Schlitze eine Entfaltung der Stützbügel 2 ermöglicht wird. Nachfolgend kann dann eine Relativbewegung der Elemente der Kartusche 6 in lateraler Richtung zu weiteren Entfaltungen von Kommissurenbügeln 3 und anderen Elementen der Verankerungsstütze 1 durchgeführt werden.

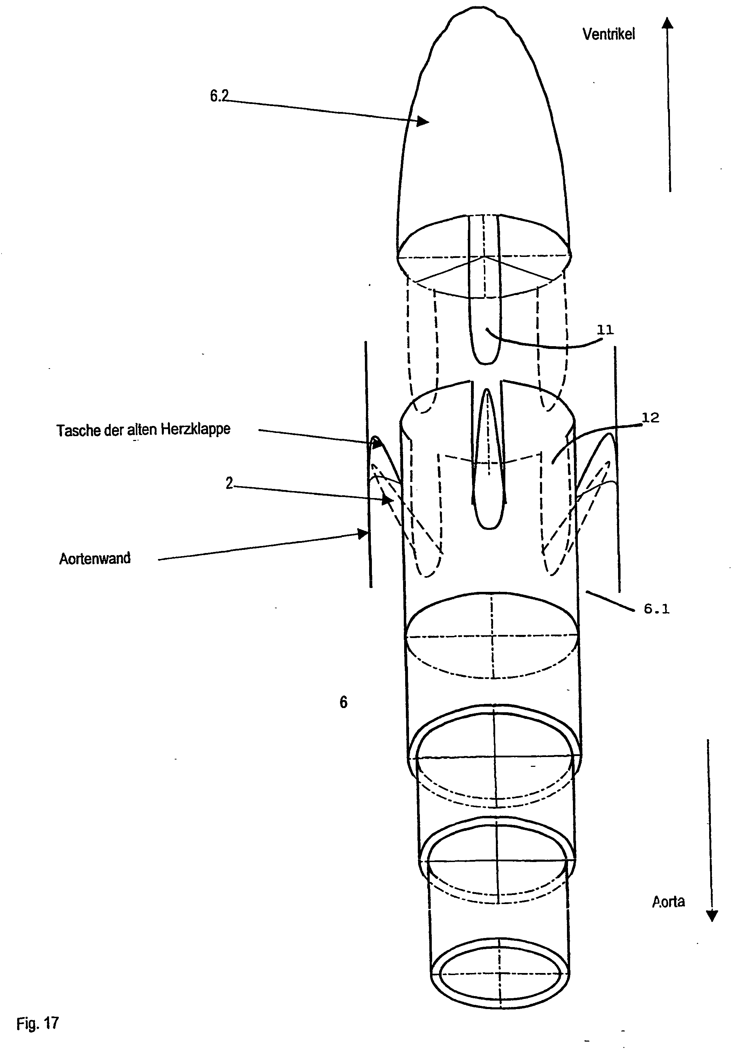

In Figur 17 ist aber gezeigt, wie eine freigebende Bewegung in proximal, lateraler Richtung des proximalen Teils 6.2 eine solche sequentielle Entfaltung ermöglicht.

Dabei sind am Teil 6.1 der Kartusche 6 ausgehend von ihrer proximalen Stirnseite in distaler Richtung Schlitze 12 ausgebildet. Die Schlitze 12 wurden vorab mit den Stegen 11 des proximalen Teiles 6.2 überdeckt, so dass auch die Stützbügel 2 innerhalb der Kartusche 6 zusammengefaltet gehalten worden sind.

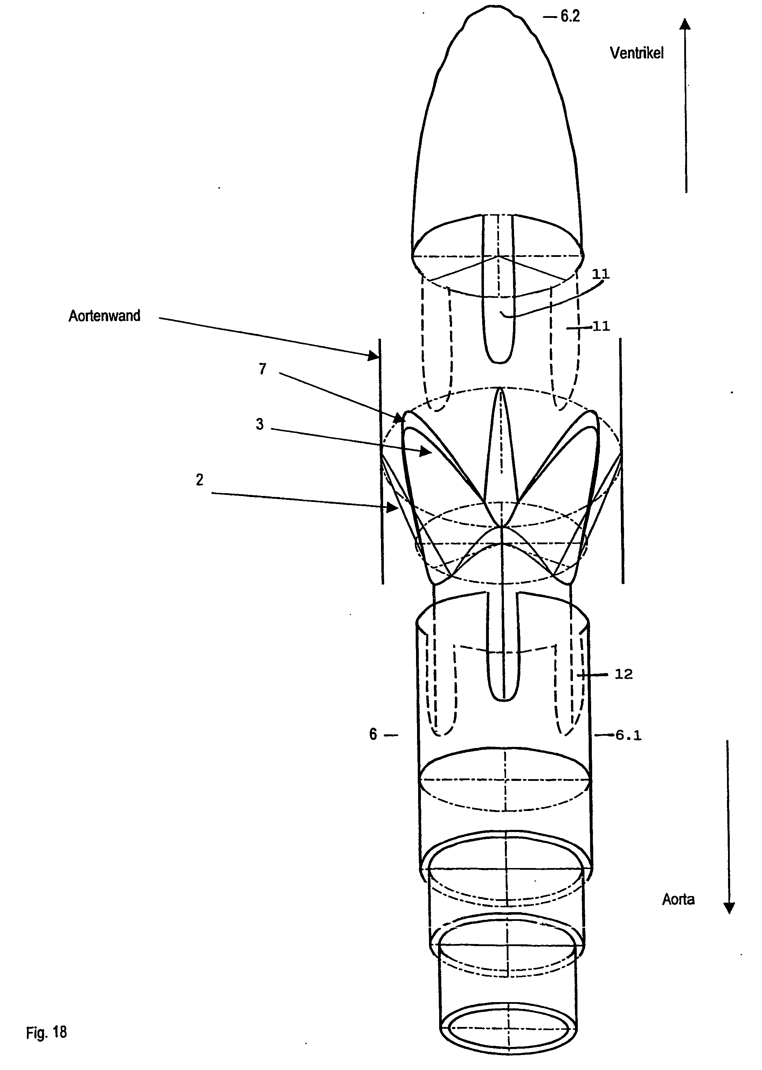

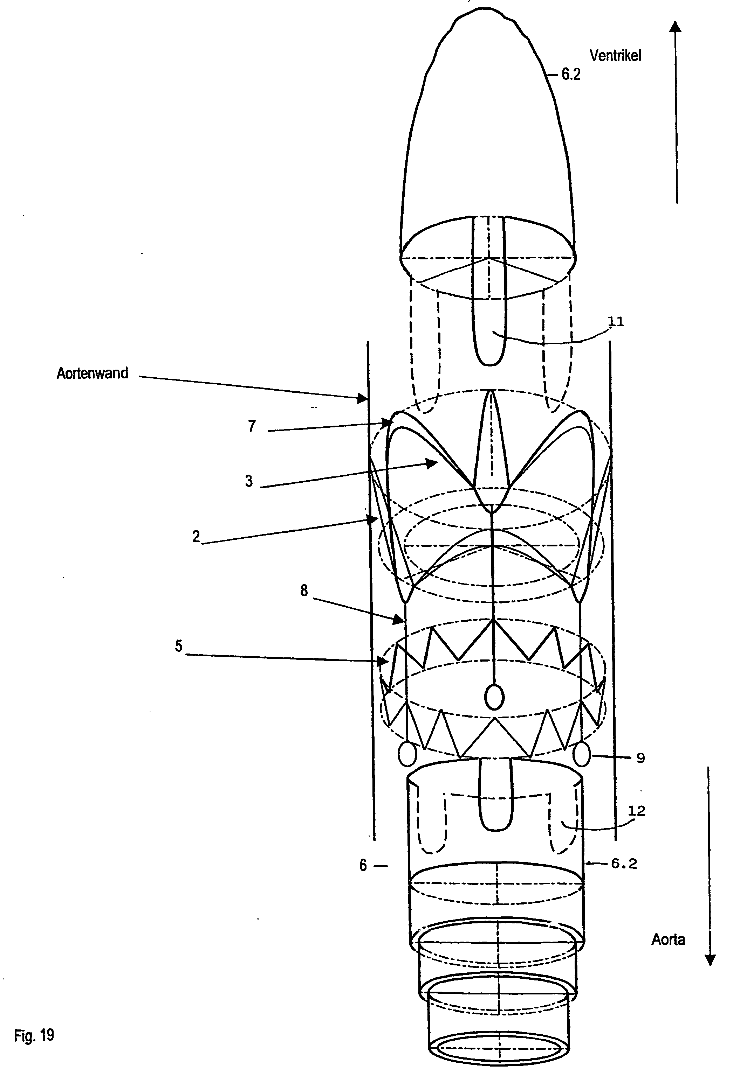

Nachdem eine Entfaltung und Einführung der Stützbügel 2 in die Tasche einer alten Herzklappe erfolgt ist, führt eine weitergehende Bewegung der Kartusche 6 auch zur Freigabe und radial nach außen wirkenden Entfaltung von Kommissurenbügeln 3 und Stützbügeln 7. Nach weiterer entsprechender Bewegung der distalen Elemente der Kartusche 6 werden alle Elemente der Verankerungsstütze zur ihrer selbstexpandierenden Entfaltung freigegeben, wie es mit den Figuren 18 und 19 in zwei Phasen einer Implantation für eine Herzklappenprothese 4 angedeutet ist.

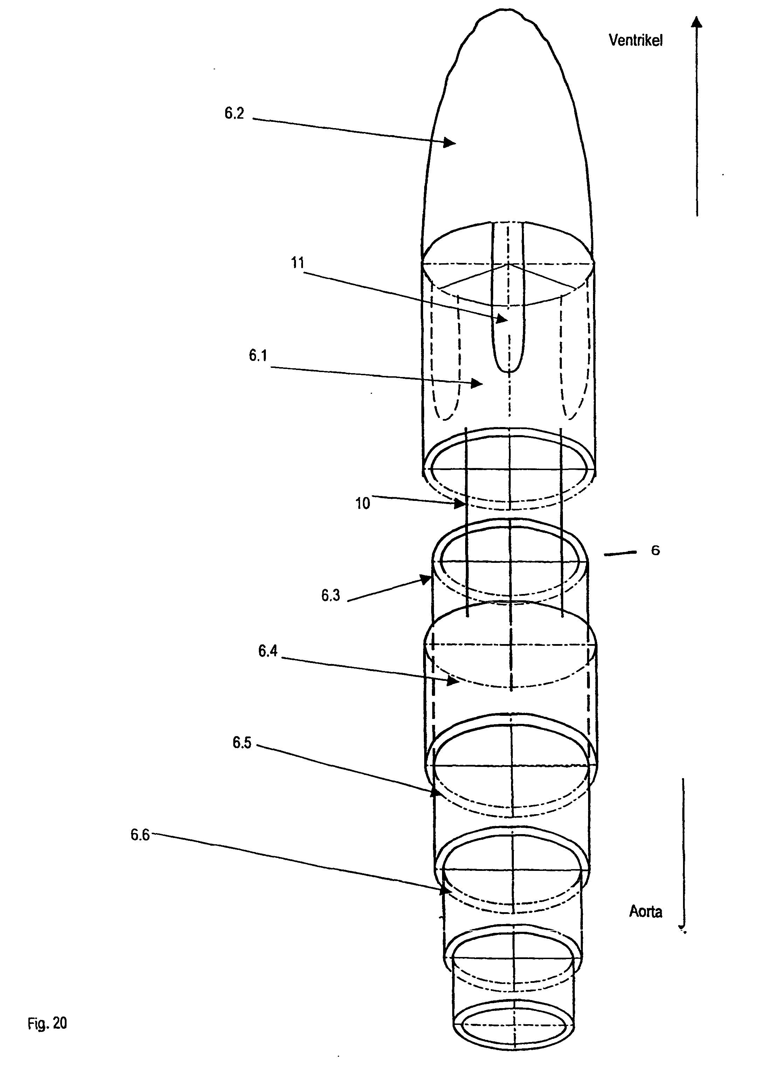

Das in Figur 20 gezeigte Beispiel einer Kartusche 6 weist analog zu dem Beispiel, wie es in den Figuren 16 bis 19 dargestellt worden ist, wieder am proximalen Teil 6.2 Stege 11 auf und es können am Teil 6.1 wieder Schlitze 12 vorhanden sein, wobei die Schlitze 12 nicht zwingend erforderlich sind.

Es sind bei diesem Beispiel wiederum Betätigungselemente 10, in Form von Bowdenzügen angedeutet.

Das Teil 6.3 der Kartusche 6 nimmt im Wesentlichen den Ringträger 5 mit entsprechend ausgebildeter Stützstruktur auf und das Teil 6.4 dient im Wesentlichen zur Steuerung des Teiles 6.1.

Die Teile 6.5 und 6.6 können als Führungen und für die Steuerung der weiteren Elemente einer Kartusche 6 für die jeweils erforderlichen Bewegungen, die zur sequentiellen Freigabe und Entfaltung von Elementen einer Verankerungsstütze 1 erforderlich sind, ermöglichen.

## Patentansprüche

1. Vorrichtung zur transvaskulären Implantation und Befestigung von Herzklappenprothesen mit einer an einer selbstexpandierbaren Verankerungsstütze befestigten Herzklappenprothese,
**dadurch gekennzeichnet, dass** die Verankerungsstütze (1) in gefalteter Form in einer mit einem Katheter verbindbaren Kartusche (6) aufgenommen ist;
proximalseitig an der Verankerungsstütze (1) Stützbügel (2) ausgebildet sind, die in Taschen der Herzklappe eines Patienten einführbar sind; und
an der Verankerungsstütze (1) zusätzlich Kommissurenbügel (3) vorhanden sind, die mit den Stützbügeln (2) Teile der Herzklappe eines Patienten, die jeweils zwischen einem Stütz- und einem Kommissurbügel (2, 3) angeordnet sind, nach erfolgter Entfaltung der Verankerungsstütze (1) klemmend halten;
dabei durch eine vorgebbare erste Bewegung der Kartusche (6) lediglich die Stützbügel (2) der Verankerungsstütze (1) zum Einführen in Taschen von Herzklappen und
durch mindestens eine zweite nachfolgende Bewegung der Kartusche (6) und/oder des Katheters weitere Elemente (3, 4, 5, 5', 7, 8, 9) der Verankerungsstütze (1) mit Herzklappenprothese (4) zu deren vollständiger Expansion und Verankerung der Verankerungsstütze (1) freigebbar sind.

2. Vorrichtung nach Anspruch 1,
**dadurch gekennzeichnet, dass** die Kartusche (6) aus mehreren Teilen (6.1 - 6.6) gebildet ist, die relativ zueinander bewegbar sind.

3. Vorrichtung nach Anspruch 1 oder 2,
**dadurch gekennzeichnet, dass** die Kartusche (6) oder einzelne Teile einer Kartusche (6.1, 6.2) in proximaler und/oder distaler Richtung verschiebbar ist/sind.

4. Vorrichtung nach einem der vorhergehenden Ansprüche, **dadurch gekennzeichnet, dass** die Kartusche (6) oder einzelne Teile von Kartuschen in Stufen verschiebbar ist/sind.

5. Vorrichtung nach einem der vorhergehenden Ansprüche, **dadurch gekennzeichnet, dass** zur Verschiebung der Kartusche (6) oder einzelnen Teile der Kartusche Bowdenzüge oder flexible Schubrohre (10) durch das Innere des Katheters zur Kartusche (6) oder einem Teil einer Kartusche (6) geführt sind.

6. Vorrichtung nach einem der vorhergehenden Ansprüche, **dadurch gekennzeichnet, dass** an der Kartusche (6) eine Entfaltung von Stützbügeln (2) ermöglichende Schlitze (12) ausgebildet sind.

7. Vorrichtung nach Anspruch 6,
**dadurch gekennzeichnet, dass** die Schlitze (12) gerade, in schräg geneigten Winkeln oder in Kurvenform ausgebildet sind.

8. Vorrichtung nach einem der vorhergehenden Ansprüche, **dadurch gekennzeichnet, dass** die Kartusche (6) oder ein Teil einer Kartusche zur Freigabe der Stützbügel (2) um ihre Längsachse verdrehbar ist.

9. Vorrichtung nach einem der vorhergehenden Ansprüche, **dadurch gekennzeichnet, dass** an der Verankerungsstütze (1) Befestigungselemente (9) für die Übertragung von Dreh-, Schub- oder Zugkräften und/oder die Fixierung der Verankerungsstütze (1) angeordnet sind.

10. Vorrichtung nach einem der vorhergehenden Ansprüche, **dadurch gekennzeichnet, dass** die Stützbügel (2) und Kommissurenbügel (3) in proximaler Richtung konvex bogenförmig gekrümmt sind.

11. Vorrichtung nach einem der vorhergehenden Ansprüche, **dadurch gekennzeichnet, dass** an der Verankerungsstütze (1) zusätzliche Stabilisierungsbügel (7) vorhanden sind.

12. Vorrichtung nach einem der vorhergehenden Ansprüche, **dadurch gekennzeichnet, dass** die Stütz- und Kommissurenbügel (2, 3) mit einem Winkelversatz zueinander an der Verankerungsstütze (1) angeordnet sind.

13. Vorrichtung nach einem der vorhergehenden Ansprüche, **dadurch gekennzeichnet, dass** an der Verankerungsstütze (1) mindestens ein Ringträger (5, 5') vorhanden ist.

14. Vorrichtung nach einem der vorhergehenden Ansprüche, **dadurch gekennzeichnet, dass** die Stützbügel (2), Kommissurenbügel (3) und/oder Stabilisierungsbügel (7) mit einem Ringträger (5') verbunden sind.

15. Vorrichtung nach einem der vorhergehenden Ansprüche, **dadurch gekennzeichnet, dass** an der Verankerungsstütze (1) ein über Längsstege (8) mit Stützbügeln (2), Kommissurenbügeln (3) und/oder Stabilisierungsbügeln (7) verbundener Ringträger (5) mit einer z-förmigen, wellenförmigen oder mäanderförmigen Stützstruktur vorhanden ist.

16. Vorrichtung nach einem der vorhergehenden Ansprüche, **dadurch gekennzeichnet, dass** mehrere Teile einer Kartusche (6.1 - 6.6) in Form eines Teleskops angeordnet sind.

17. Vorrichtung nach einem der vorhergehenden Ansprüche, **dadurch gekennzeichnet, dass** an einem Teil einer Kartusche (6) Schlitze (12) und an einem zweiten relativ zu diesem Teil einer Kartusche (6) bewegbaren Teil die Schlitze (12) überdeckende Stege (11) ausgebildet sind, die eine Freigabe der Stützbügel (2) bis zum Einführen in die Taschen einer Herzklappe eines Patienten verhindern.

18. Vorrichtung nach einem der vorhergehenden Ansprüche, **dadurch gekennzeichnet, dass** an einem Teil einer Kartusche (6.2) in distaler Richtung überstehende Stege (11) ausgebildet sind.

## Claims

1. Device for the transvascular implantation and fixing of heart valve prostheses, with a heart valve prosthesis fixed to a self-expandable anchoring support, **characterized in that** the anchoring support (1) is accommodated, in a folded format, in a cartridge (6) which can be connected to a catheter;
support hoops (2) are formed at the proximal end of the anchoring support (1) and can be introduced into pockets of a patient's heart valve; and
commissural hoops (3) are additionally provided on the anchoring support (1) and, after the anchoring support (1) has been deployed, these commissural hoops (3), along with the support hoops (2), securely hold parts of a patient's heart valve which are each arranged between a support hoop and a commissural hoop (2, 3);
by means of a predetermined first movement of the cartridge (6), it is possible for just the support hoops (2) of the anchoring support (1) to be released for introduction into pockets of heart valves, and
by means of at least a second subsequent movement of the cartridge (6) and/or of the catheter, it is possible for further elements (3, 4, 5, 5', 7, 8, 9) of the anchoring support (1) with heart valve prosthesis (4) to be released in order to permit complete expansion of the latter and anchoring of the anchoring support (1).

2. Device according to Claim 1, **characterized in that** the cartridge (6) is formed from a plurality of parts (6.1 - 6.6) which are able to move relative to one another.

3. Device according to Claim 1 or 2, **characterized in that** the cartridge (6) or individual parts of a cartridge (6.1, 6.2) is/are movable in the proximal and/or distal direction.

4. Device according to one of the preceding claims, **characterized in that** the cartridge (6) or individual parts of cartridges is/are movable in stages.

5. Device according to one of the preceding claims, **characterized in that**, in order to move the cartridge (6) or individual parts of the cartridge, Bowden wires or flexible pusher tubes (10) are guided through the inside of the catheter to the cartridge (6) or to a part of a cartridge (6).

6. Device according to one of the preceding claims, **characterized in that** slits (12) permitting deployment of support hoops (2) are formed on the cartridge (6).

7. Device according to Claim 6, **characterized in that** the slits (12) are configured as straight lines, at obliquely inclined angles or in a curve shape.

8. Device according to one of the preceding claims, **characterized in that** the cartridge (6) or a part of a cartridge can turn about its longitudinal axis in order to release the support hoops (2).

9. Device according to one of the preceding claims, **characterized in that** fastening elements (9) are arranged on the anchoring support (1) for the purpose of transmitting rotation forces, pushing forces or tensile forces and/or for fixing the anchoring support (1).

10. Device according to one of the preceding claims, **characterized in that** the support hoops (2) and the commissural hoops (3) are curved in a convex arc in the proximal direction.

11. Device according to one of the preceding claims, **characterized in that** additional stabilizing hoops (7) are provided on the anchoring support (1).

12. Device according to one of the preceding claims, **characterized in that** the support hoops and commissural hoops (2, 3) are arranged offset at an angle to each other on the anchoring support (1).

13. Device according to one of the preceding claims, **characterized in that** at least one annular carrier (5, 5') is provided on the anchoring support (1).

14. Device according to one of the preceding claims, **characterized in that** the support hoops (2), the commissural hoops (3) and/or the stabilizing hoops (7) are connected to an annular carrier (5').

15. Device according to one of the preceding claims, **characterized in that** an annular carrier (5) which has a z-shaped, undulating or meandering support structure and can be connected via lengthwise webs (8) to support hoops (2), commissural hoops (3) and/or stabilizing hoops (7) is provided on the anchoring support (1).

16. Device according to one of the preceding claims, **characterized in that** a plurality of parts of a cartridge (6.1 - 6.6) are arranged in the form of a telescope.

17. Device according to one of the preceding claims, **characterized in that** one part of a cartridge (6) is provided with slits (12), and a second part movable relative to the first part of a cartridge (6) is provided with webs (11) which cover the slits (12) and prevent release of the support hoops (2) before introduction into the pockets of a patient's heart valve.

18. Device according to one of the preceding claims, **characterized in that** webs (11) protruding in the distal direction are formed on a cartridge part (6.2).

## Revendications

1. Dispositif pour l'implantation transvasculaire et la fixation des prothèses de valvules cardiaques avec une prothèse de valvule cardiaque fixée sur un support d'ancrage autoexpansible,
**caractérisé en ce que** le support d'ancrage (1) est réceptionné dans une forme pliée dans une cartouche (6) pouvant être reliée à un cathéter ;
des étriers de support (2), qui peuvent être introduits dans des poches de la valvule cardiaque d'un patient, sont réalisés du côté proximal sur le support d'ancrage (1), et
des étriers à commissures (3) sont présents en supplément sur le support d'ancrage (1), lesquels maintiennent par serrage avec les étriers de support (2) des parties de la valvule cardiaque d'un patient, qui sont disposées respectivement entre un étrier de support et un étrier à commissure (2, 3), après le déploiement du support d'ancrage (1) ;
seuls les étriers de support (2) du support d'ancrage (1) peuvent être libérés par un premier déplacement prédéfinissable de la cartouche (6) pour l'introduction dans des poches de valvules cardiaques et
d'autres éléments (3, 4, 5, 5', 7, 8, 9) du support d'ancrage (1) avec une prothèse de valvule cardiaque (4) peuvent être libérés par au moins un second déplacement consécutif de la cartouche (6) et/ou du cathéter pour leur expansion complète et l'ancrage du support d'ancrage (1).

2. Dispositif selon la revendication 1,
**caractérisé en ce que** la cartouche (6) est formée de plusieurs parties (6.1 - 6.6) qui peuvent être déplacées les unes par rapport aux autres.

3. Dispositif selon la revendication 1 ou 2,
**caractérisé en ce que** la cartouche (6) ou des parties individuelles d'une cartouche (6.1, 6.2) est/sont mobiles en coulissement dans une direction proximale et/ou une direction distale.

4. Dispositif selon l'une quelconque des revendications précédentes, **caractérisé en ce que** la cartouche (6) ou des parties individuelles de cartouches est/sont mobiles en coulissement dans des niveaux.

5. Dispositif selon l'une quelconque des revendications précédentes, **caractérisé en ce que**, pour le coulissement de la cartouche (6) ou de parties individuelles de la cartouche, des câbles sous gaine ou des tubes de poussée (10) souples sont guidés à travers l'intérieur du cathéter vers la cartouche (6) ou une partie d'une cartouche (6).

6. Dispositif selon l'une quelconque des revendications précédentes, **caractérisé en ce que** des fentes (12) permettant un déploiement d'étriers de support (2) sont réalisées sur la cartouche (6).

7. Dispositif selon la revendication 6,
**caractérisé en ce que** les fentes (12) sont conçues droites, dans des angles inclinés de façon oblique ou en forme de came.

8. Dispositif selon l'une quelconque des revendications précédentes, **caractérisé en ce que** la cartouche (6) ou une partie d'une cartouche peut tourner autour de son axe longitudinal pour libérer les étriers de support (2).

9. Dispositif selon l'une quelconque des revendications précédentes, **caractérisé en ce que** sur le support d'ancrage (1) sont disposés des éléments de fixation (9) pour la transmission de forces de rotation, de poussée ou de traction et/ou la fixation du support d'ancrage (1).

10. Dispositif selon l'une quelconque des revendications précédentes, **caractérisé en ce que** les étriers de support (2) et les étriers à commissures (3) sont incurvés de façon convexe en forme d'arc dans le sens proximal.

11. Dispositif selon l'une quelconque des revendications précédentes, **caractérisé en ce que** des étriers de stabilisation (7) supplémentaires sont présents sur le support d'ancrage (1).

12. Dispositif selon l'une quelconque des revendications précédentes, **caractérisé en ce que** les étriers de support et les étriers à commissures (2, 3) sont disposés avec un déport d'angle les uns par rapport aux autres sur le support d'ancrage (1).

13. Dispositif selon l'une quelconque des revendications précédentes, **caractérisé en ce qu'**au moins un support annulaire (5, 5') est présent sur le support d'ancrage (1).

14. Dispositif selon l'une quelconque des revendications précédentes, **caractérisé en ce que** les étriers de support (2), les étriers à commissures (3) et/ou les étriers de stabilisation (7) sont reliés à un support annulaire (5').

15. Dispositif selon l'une quelconque des revendications précédentes, **caractérisé en ce qu'**un support annulaire (5) relié au moyen de barrettes longitudinales (8) à des étriers de support (2), des étriers à commissures (3) et/ou des étriers de stabilisation (7) avec une structure de soutien en forme de z, de forme ondulée ou en forme de méandre est présent sur le support d'ancrage (1).

16. Dispositif selon l'une quelconque des revendications précédentes, **caractérisé en ce que** plusieurs parties d'une cartouche (6.1 - 6.6) sont disposées sous la forme d'un télescope.

17. Dispositif selon l'une quelconque des revendications précédentes, **caractérisé en ce que** sur une partie d'une cartouche (6) sont conçues des fentes (12) et sur une seconde partie, pouvant être déplacée par rapport à cette partie d'une cartouche (6), sont réalisées des barrettes (11) recouvrant les fentes (12), qui empêchent une libération des étriers de support (2) jusqu'à l'introduction dans des poches d'une valvule cardiaque d'un patient.

18. Dispositif selon l'une quelconque des revendications précédentes, **caractérisé en ce que** des barrettes (11) saillantes dans le sens distal sont conçues sur une partie d'une cartouche (6.2).
